# EUROPEAN PATENT SPECIFICATION

(11) **EP 1 667 589 B1**
(45) Date of publication and mention of the grant of the patent: **25.11.2009**
(21) Application number: 04768433.7
(22) Date of filing: 10.09.2004
(51) Int. Cl.: A61B 17/43

(54) **APPARATUS FOR HANDLING CELLS, EMBRYOS OR OOCYTES**
GERÄT ZUR HANDHABUNG VON ZELLEN, EMBRYOS ODER OOZYTEN
APPAREIL ET PROCEDE DE MANIPULATION DE CELLULES, D'EMBRYONS OU D'OVOCYTES

(30) Priority: 10.09.2003 GB 0321158
(43) Date of publication of application: 14.06.2006
(73) Proprietor: Overture Research Inc., Mill Valley, CA 94941-1554 (US)
(72) Inventor: DODGSON, John, Robert, London N19 5RZ (GB)
(74) Representative: Sharp, Alan Cooper
(86) International application number: PCT/GB2004/003886
(87) International publication number: WO 2005/023124

(56) References cited:
- EP-A- 1 340 810
- WO-A2-01/88087
- US-A1- 2003 107 386
- US-B1- 6 184 029

## Description

This invention relates to apparatus and methods for handling of cells and other cellular entities, in particular oocytes and embryos.

It is known to manipulate single cells using pipettes etc. and to isolate single cells in wells in an array format. The wells might be much larger than the cell, or of a similar size. Cell holding arrays in sieve-like or filter-like substrates are known, with the cell holding positions in a regular array or a random pattern. Cells are typically positioned using suction, giving liquid flow through the substrate, but other forces, such as electrophoresis or dielectrophoresis or sedimentation under gravity are also used in the prior art. Using present array-based devices it is difficult to control precisely the liquid conditions around the cells, while minimising the amount of liquid needed. This is a disadvantage when the liquid is precious or if gradual changes or patterns of change of liquid are needed, as encountered for example in maturation of oocytes or embryos.

US 6,193,647 discloses a network of microfluidic channels to handle embryos- these are entrained in flow and moved in the liquid through 'embryo transport channels', and held at required positions using 'formations' in the channel, in particular at a constriction. This gives easy insertion and retrieval of a single embryo from a given channel, but is less advantageous if more then one embryo is present in the channel.

It is known to be advantageous in certain applications to culture oocytes and embryos in groups. A number of embryos can be contained in the same channel but it is hard to access a particular one. No means is disclosed in US 6,193,647 to select a single embryo from a given group. In the embodiments shown in US 6,193,647 a relatively large amount of solution is needed to bathe and exchange solution over a given oocyte. The device disclosed in US 6,193,647 has a number of drawbacks. For example, it requires relatively complex assembly for mass production of the embryo device - e.g. microfabrication in silicon. The device is also not adapted to implement easily robotic insertion and retrieval of embryos.

US 6,184,029 describes a mesoscale sample preparation apparatus for preparing a cell enriched fraction of a sample for performing analysis thereon.

The present invention provides an improved apparatus for handling cellular entities such as cells, oocytes and embryos which allows easy access to individual cellular entities in a group, while being able easily to expose the group to common liquid conditions, for example to programme their development or metabolism or to conduct test procedures on them. The invention aims to combine the advantages of the microfluidic approach, in which cellular entities can be held at a given position within a device while flow of liquid can be maintained in their vicinity, with the easy manipulation allowed by an array format in which the cellular entities are held in preformed wells which are in communication with microfluidic channels.

The cellular entities capable of being handled by the present invention include oocytes, embryos or other complexes of cells, individual cells themselves and groups of cells of single or mixed type. Typical cells will be smaller than oocytes or embryos, but the same principles apply on a smaller size scale. The terms oocyte, embryo, cell and cellular entity are used interchangeably in the following description.

According to a first aspect of the present invention there is provided a device as specified in claims 1 to 17. According to a second aspect of the present invention, there is provided a method of culturing a cellular entity as specified in claims 18 or 19.

The invention provides an apparatus and a method to handle one or more oocytes or cellular entities in parallel using conventional fluidic robotics. The apparatus is able to:-
select and handle individual oocytes with minimal disturbance to others, while allowing multiple oocytes to be exposed to common fluidic conditions
allow ready exchange of medium over individual or groups of oocytes
in certain embodiments, achieve good visibility of the oocytes to allow visual assessment and selection in-situ
isolate a given containment component from the apparatus for transportation or storage while maintaining controlled conditions within the oocyte environment,
allow the disposable parts of the apparatus to be made cheaply in bulk.

The apparatus and method can be used, for example, in culturing and maturation of embryos and oocytes in procedures such as cloning or in-vitro fertilisation, culturing and maturation of single cells and groups of cells in stem cell or other research.

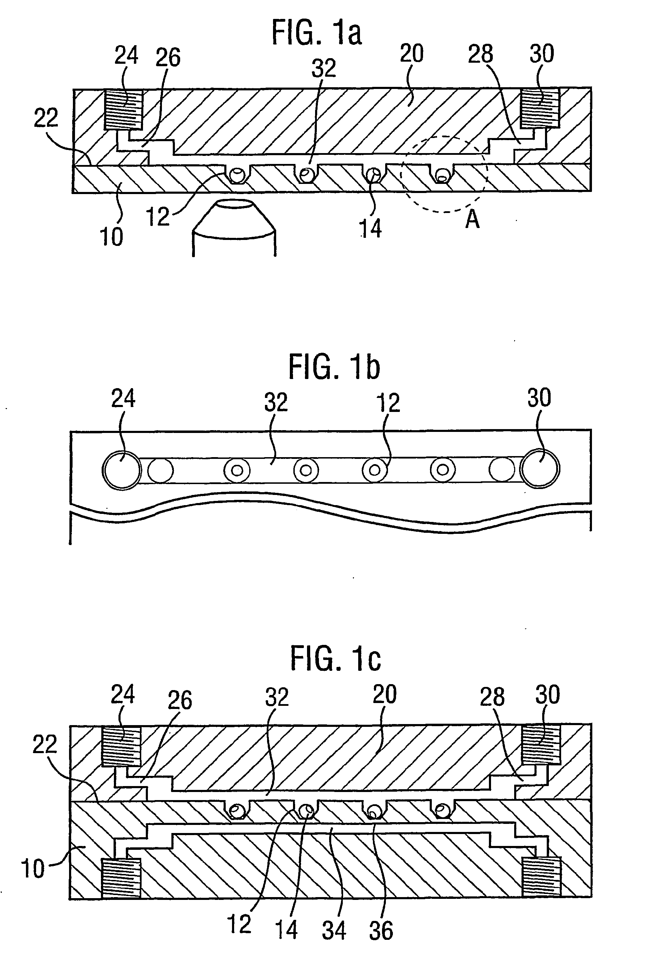

Embodiments of the invention will now be described, by way of example only, with reference to the accompanying diagrammatic figures in which:-
Figures 1 a and b show a cross-section and plan view of a first apparatus according to the invention,
Figure 1 c shows a cross section of an alternative embodiment,
Figure 2 shows cross sections of preferred well shapes,
Figure 3 shows how liquid in the apparatus is moved using air pressure,
Figures 4a - o show alternative embodiments according to the invention in cross-section and plan views,
Figures 5 and 6 show further embodiments of the present invention in cross-section,
Figure 7 shows an oblique view of a robotic pipette with an apparatus according to the present invention,
Figure 8 shows an alternative embodiment in cross-section having first and second substrates assembled together,
Figure 9 shows the embodiment of Figure 8 with first and second substrates separated,
Figure 10 shows a plan view of the an apparatus when assembled,
Figure 11 shows a cross-section of the apparatus of Figure 10 along the section at X-X, and
Figure 12 a cross-section of the apparatus of Figure 10 along the section at Y-Y.

### Example 1

Figure 1 shows an apparatus according to the present invention in which a first substrate (10) is provided with an array of first wells (12) open to a major surface of said substrate. Each well 12 is adapted to hold oocytes or other cellular entities (14). In the present embodiment the wells are tapered to locate the cellular entity at a specific location in each well. The apparatus also comprises one or more fluidic channels (32) open to each well.

In the embodiment shown in Figure 1, the fluidic channel is formed on the major surface of a further substrate (20), which surface is adapted and arranged to face the first substrate carrying the array of wells. This further substrate in the present embodiment forms a lid, which seals to the substrate at a sealing surface (22), the lid comprising an inlet channel port 24, an inlet channel 26, an outlet channel 28 and an outlet port 30; also comprising a channel formation which when sealed against the substrate forms a channel 32 through which fluid can flow from the inlet port to the outlet port, in communication with one or more of the wells 12. It will be understood that the arrangement of liquid connections shown in fig. 1a is diagrammatic and connections to the channels can be made in any manner as known in the art.

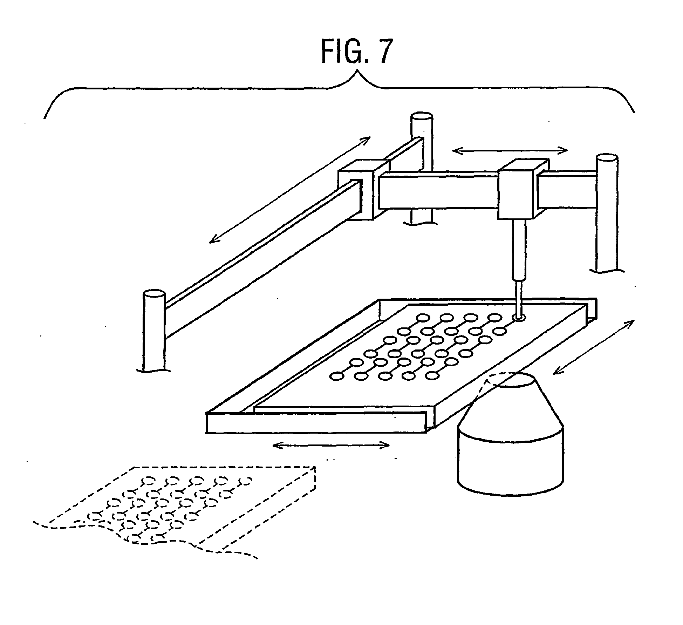

The substrate is preferably transparent and the wells 12 are preferably adapted to give good visibility from below using an inverted microscope. Alternatively, the lid 20 is transparent, and the upper surface of the channel formation 32 in the lid is of good optical quality so that the cellular entities can be observed from above. The substrate and wells are designed to allow access from above using a pipette. The wells are optionally spaced regularly in a 1D or a 2D array, for example at positions according to the SBS microplate standard to allow ready interface to a robotic pipettor, as shown in Figure 7.

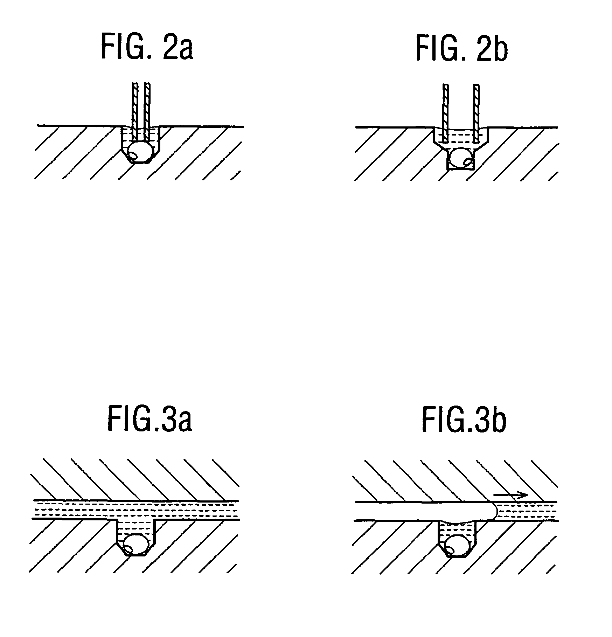

The wells 12 are preferably tapered, to allow easy access into the well from above, while locating the cellular entities in a small area of the well base. The wells can be dimensioned such that they are close in size to the diameter of the cellular entity 14, as shown in Figure 2a: in this case a small diameter pipette will be needed to handle the entity - in handling, they then will not be entrained in liquid aspirated into the pipette, but instead will be held against the end of the pipette as in conventional usage of a holding pipette. Preferably the wells are sized to allow a pipette to be used of sufficient internal diameter to entrain the entities into the pipette along with surrounding medium. The well will then preferably taper to a holding position for the cellular entity close to its diameter, as shown in Figure 2b. The cellular entities are accommodated in the base of the wells, which are so sized that the cellular entities will not be affected by flow through the channel 32. The channel 32 may also be sized to prevent cellular entities from passing through it.

In the embodiment in fig. 1a the channel 32 is shown as being formed in the surface of the lid. In an alternative embodiment, the channel 32 can be formed in the surface of the substrate 10, the lid then having a flat profile over the position of the wells.

The sealing surface 22 between the lid and the substrate needs to define a sealed channel between the two. The seal might be achieved using a compliant surface 22, for example a gasket mounted on the substrate or the lid; alternatively the lid might be formed partially or entirely of a compliant material such as PDMS. The seal might be formed using hydrophobicity of the seal region, such that solution in the channel 32 does not spread to wet the seal surface 22. The hydrophobic surface might be achieved using hydrophobic materials for substrate or lid or both, a hydrophobic gasket or coating on one or both surfaces.

The lid is preferably clamped to the substrate using a clamping means (not shown) that locates the lid and the substrate one relative to another and achieves a seal and holds the lid stationary relative to the substrate.

In an alternative embodiment (not shown) the solution might wet the surfaces 22, a degree of wetting between them being acceptable in the design in that exchange of components between the film of liquid in the seal region, and liquid flowing through the channel, can be accepted as negligible.

The method in which the apparatus of Figure 1 is used is as follows.

With the lid removed, the substrate is placed in an automatic pipettor, and cellular entities are dispensed, one to a well, together with a volume of medium that substantially fills them. The pipette is sized so as to hold the cellular entity within it. The cellular entities then sediment to the holding position in the base of the well. The lid is then fitted and held in place using a clamping mechanism (not shown) and liquid is flowed through the channel 32, contacting the menisci in the wells.

Contents of the wells are not actively flowed out of the well; compounds exchange between the channel and the wells by diffusion. The wells and the channels are scaled so that this exchange will take place appropriately quickly. In general in apparatus for oocyte or embryo maturation rapid changes of chemical environment are not required (and generally are to be avoided), so the required diffusion times and hence dimensions of the channel and well are not too small.

Typical diffusion times and distances are related by Dt/1¹ ~ 1 for complete equilibration. For a typical small protein, which is the largest molecule in a typical maturation medium, D ~ 10⁻⁶ cm²s⁻¹, giving an equilibration time of 100s for a 100 µm deep well and 400s for a 200 µm well. (Almost) complete exchange of one protein for another will take longer, corresponding to Dt/l² of around 5. These times are long, but not too long for applications in oocyte maturation.

The channel 32 is preferably emptied before the lid is removed from the substrate. In the present embodiment this is achieved by forcing gas through the channel, so displacing liquid through the outlet. The wells have no outlet, so the liquid level in the wells is negligibly affected as shown in Figure 3a and 3b. The lid is then free of solution and can be removed, leaving the cellular entity environment in the wells unaffected.

The handling system preferably comprises means for tracking the status of the cellular entity in each well, and the application of compounds to them. It might then interface with an automatic microscope stage and a data-entry system to correlate observations made on each cellular entity with their position, so allowing easy data handling and tracking of experiments.

Each well is usually intended to receive a single cellular entity, though some instances may favour more than one in each. The wells are dimensioned such that a cellular entity can be dispensed into the well from a pipette, and will sediment under gravity or move entrained in liquid towards a holding position in the well. The cellular entity is not transported with a rolling motion by liquid flow in a channel in the sense of US 6,193,647 - rather it is dispensed into a well similar to those known in the art, but with improved features rendering it suitable for retention of a cellular entity in a chosen position within it and to give easy exchange of liquid surrounding the cellular entity.

In oocyte culture it is necessary to maintain the contents of the medium within certain limits. In general the medium is ideally in equilibrium with 5% CO2 in air; metabolism by the oocyte means that gas is required to be supplied to the medium in the vicinity of the oocyte. In conventional culture this occurs by diffusion through the medium from the incubator atmosphere. In the apparatus of the invention, gas diffuses from the medium in the front side channel into and out of the wells. Particularly if the medium is stationary for a period of time, it is advantageous in some applications to ensure the constant supply of gas to the wells. This is achieved in the embodiment shown in fig. 1c, in which the lid 20 is the same as in fig. 1a; however the substrate 10 comprises additionally a gas supply channel 34 running close to the base (or side) of the wells, separated from the well interior by a gas-permeable material. This is achieved simply in practice by fabricating the well base, or the whole of the substrate 10, from a gas-permeable polymer such as PDMS. The well base might be formed by a layer of gas-permeable material laminated into the structure of the substrate. Equally, a porous hydrophobic material such as porous fluorocarbon film might be used in a laminated construction to form the well base.

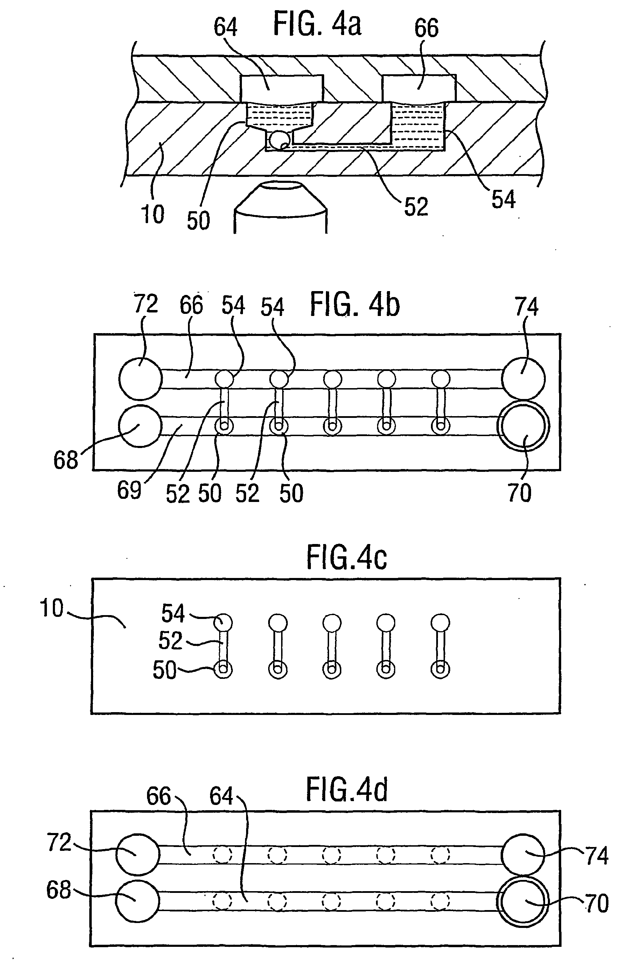

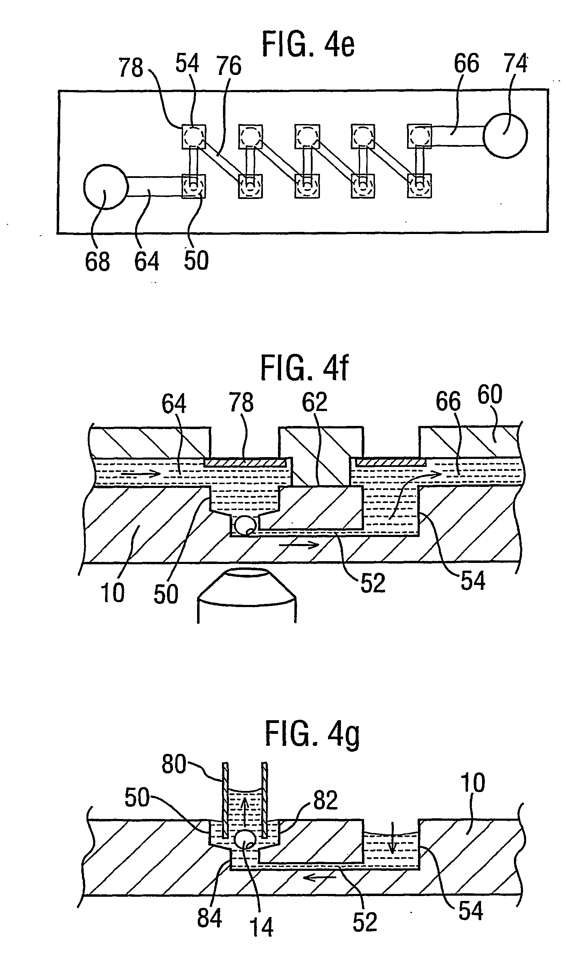

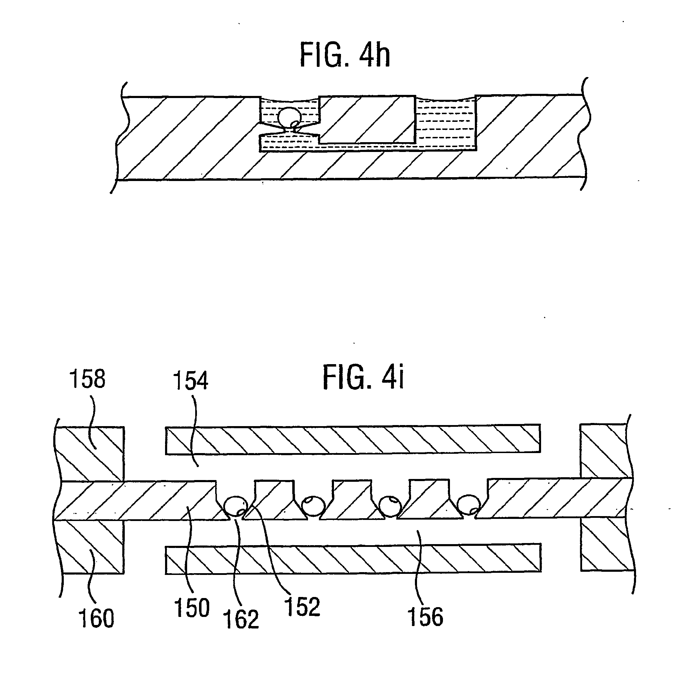

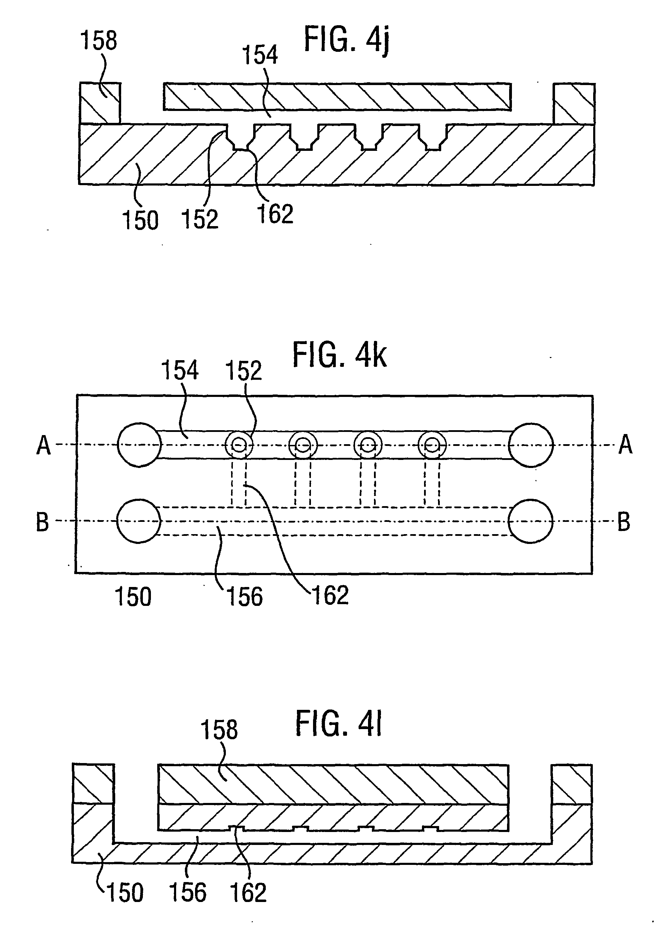

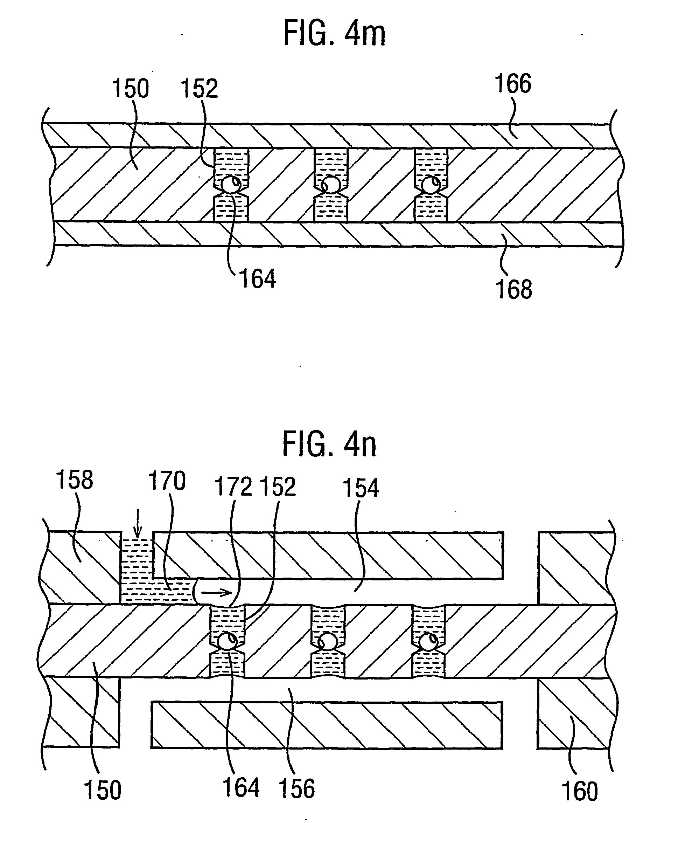

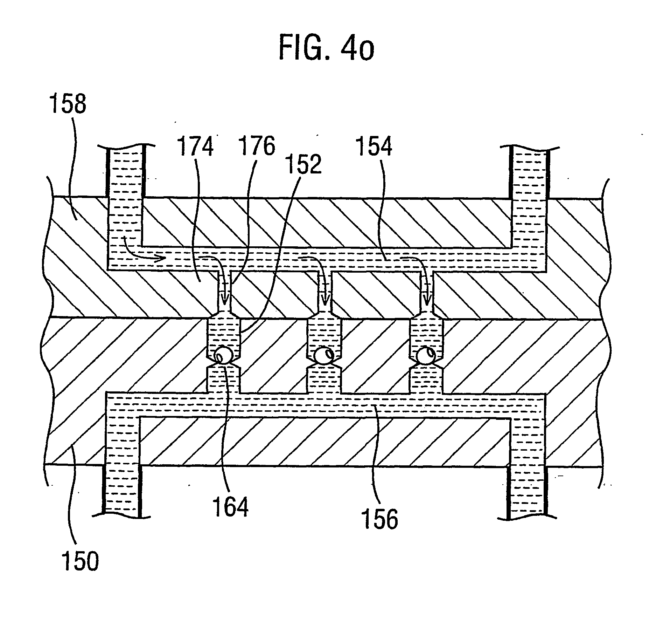

In another embodiment, the well is equipped with a channel leading from its base to a second well, as shown in Figure 4, or to a channel which acts to hold liquid which can flow into it from the first well, or can be dispensed into it from above. At least the first well is designed also to have its contents aspirated into a pipette. The second well acts to provide a flow of liquid from the second to the first well during aspiration, this flow acting to carry the cellular entity from the well into the pipette.

Observation of the cellular entity might be done from below or from above. The holding position for the cellular entity in the well is preferably at the base of the well, and the material of the substrate is transparent, so allowing ready visibility of the cellular entity from below using an inverted microscope. Alternatively, the holding position of the cellular entity might be at a height above the base, with a constriction acting to locate the cellular entity. A cover may be provided to reduce evaporation and optionally to define the volume of the well - the liquid contents of the well might in use sit below the level of the lid, or might contact the lid. This latter arrangement is advantageous in the case of observation from above so as to provide an optically clear interface.

The wells may be separate or may be linked with one or more common fluidic channels, so allowing common supply of liquid to the wells. Flow of liquid to and from the wells might be entirely by means of pipetting from above, or a combination of this and flow through channels communicating with the wells. In some embodiments gas pressure may be used to transport liquid between the first and the second well, between either or both of these and a common channel. In this way, liquid can be dispensed into one or more wells on the substrate and then moved on the substrate by external gas pressure. The gas pressure might be positive or negative. In particular, negative pressure might be used, exerted on the second well, to cause liquid to flow from the first to the second well, either to locate the cellular entity at the holding position or to cause flow of liquid past the cellular entity while at that position.

The wells are preferably arranged on the substrate so as to facilitate the robotic handling of liquids and cellular entities. For example the wells might be located at the well positions on an SBS standard 1536 well plate. This will provide a lower density of cellular entities on a plate than is feasible from the required dimensions of the wells, but does allow easy interfacing to external robotics. The density of the wells might be higher if more precise liquid handling equipment is used.

Fluid connection to the substrate might be by pipette alone, i.e. there are no physical connections to the substrate through which fluids flow, or there might be such connections to one or more locations on the substrate. Fluid connections might be made to the lower side of the substrate, or to regions of the top side of the substrate beside and clear of the pipette access region, connection to the wells being made by channels within the substrate, or both of these. Alternatively the substrate might have connections made by moveable components which are in contact with the substrate when connection is needed, and are removed when it is not, for example when access is needed to the upper surface for pipetting. The flow channels to the wells might be formed in the connection components, which in some embodiments then could act to programme the fluidic connections on the substrate so as to connect certain wells and channels at one point of the operating cycle and others at other points. Such connection components will comprise compliant parts to achieve fluid-tight seals in contact with the substrate.

The substrate is preferably used as part of a handling system which plans the sequence of pipette additions of liquid to each well to achieve a desired sequence of changes to the cellular entity environment. The system preferably couples to an automatic microscope stage so allowing easy recording of observations of the cellular entities. In some of the embodiments, observations are made from above, and the lid component may need to be in place to give a defined liquid interface for good visibility. The system then stores decisions on cellular entity handling for later execution when the lid is removed. A layout of the wells in a regular array assists this - the use of such a system will allow a large number of cellular entities to be handled on a single substrate while minimising the need for operator effort.

### Example 2

Figs. 4a - 4h show partial views of a well structure forming part of a substrate according to Figure 1. The embodiment in Fig. 4a comprises a substrate 10 comprising one or more first wells 50 adapted to receive a cellular entity, each in communication via a channel 52 having a second well 54. The channel is dimensioned such that the cellular entity intended to be confined in the first well cannot pass through it. A lid 60 seals to the substrate at a seal surface 62, and comprises one or more flow channels (64, 66) which communicate with the first and second wells. It is a feature of this embodiment that the flow pattern in the wells is determined by the arrangement of channels in the lid. Fig. 4a shows an arrangement in which the lid channels are shown to run perpendicular to the plane of the paper and might communicate with more than one of the first and the second wells. This arrangement is shown in plan view in Fig. 4b, in which a plurality of first wells 50 and second wells 54 are connected in parallel to inlet channels 64 and outlet channels 66 respectively. The substrate 10 in this embodiment is shown in Fig. 4c and the lid in Fig. 4d.

In an alternative embodiment, one or both of the channels 64 and 66 are formed in the surface of the substrate 10, linking the first and the second wells respectively. The lid 60 then has a flat profile at the position of the wells. In this embodiment the fluidic connection to and between the wells is fixed by the design of the substrate rather than that of the lid. This embodiment allows a simpler design of lid and relaxes some constraints on alignment between the lid and the substrate when these are joined.

An alternative arrangement of channels in the lid is shown in plan in Fig. 4e and in cross-section in Fig. 4f. Channels 76 connect the second well 54 of the first pair of wells to the first well 50 of a second pair, so connecting the wells in series from the inlet port 68 to the outlet port 74. This embodiment requires provision of a means to remove air pockets from the system, formed when the lid 20 is fitted to the substrate, which has slugs of liquid in each well each with an air space above it. This is achieved by means of gas-permeable regions 78 in the channel in the lid, preferably above the wells. When the system is first pressurised, air is forced out through these regions, allowing liquid to form a continuous path through the system.

In use, cellular entities are pipetted into the first wells in the substrate, the lid is fitted and then medium is flowed from the inlet well 68 through the inlet channel 64 to the first wells.

The second wells 54 might be of smaller diameter than the first wells, or might be the same size or larger in the case where they allow pipette dispensing or aspiration, or act as simple overflow wells without being connected to an outlet channel. In some embodiments they may be vented through the lid to allow escape of air as they fill.

The usefulness of the paired well concept of the invention is illustrated in Fig. 4g. The first well 50 is advantageously adapted to hold the cellular entity 14 in a confined position in the well, so as to give easy location for microscope observation. Ideally the position is well localised in x, y, and z dimensions, so that an automated stage will carry the cellular entity into the field of view at high magnification and approximately into focus. This can be achieved if the base of the well 50 has a region 84 of smaller diameter than the upper part 82, which is advantageously larger to allow access by a pipette 80. The confinement 84 will not be easily flushed of liquid if there is no flow through it: that is achieved using the channel 52 and second well 54.

The first well 50 can be configured in a variety of ways, having the common feature that the cellular entity is held in a defined position and can be restrained against flow from the first well to the second. In Fig. 4h an alternative embodiment is shown in which a constriction 86 is provided within the well rather than at its base - this has the advantage that the cellular entity is centred onto the constriction by the flow, and so the well can be significantly larger than the cellular entity and still achieve precise localisation. This allows the apparatus of the invention to be applied easily also to cells smaller than oocytes, such as mammalian cells.

The well 50 and the constriction 86 can be fabricated by any means known in the art, such as moulding, embossing, laser drilling, conventional drilling. Alternatively the constriction can be formed in a component such as a silicon die, in which through holes can be formed by means known in the art, which is mounted into the substrate that defines the well. The nature of the constriction defines the way in which the oocyte or cell is observed - if it is opaque then observation will be from above, in which case the apparatus of the invention, comprising a lid and substrate is particularly advantageous: the lid comprising the microfluidic feed channels can be removed, and an optically transparent lid mounted in its place during observation.

The channel 52 in the embodiments in figs. 4a-4h might be formed by any appropriate means known in the art, such as moulding, embossing, laser drilling or ablation, or lamination of appropriate layers to define the structure. The channel might be of uniform cross section between the wells, or preferably has a portion of smaller minimum dimension adjacent the well to retain the cellular entity, with larger dimensions elsewhere, so as to minimise flow resistance. A preferred means of forming the channel is to laminate a layer of porous material between two non-porous layers -the first (oocyte containing) well might itself be partially a cut-out region in a layer of porous material, the other edge of the material communicating with a larger channel leading to the second well.

In a further preferred embodiment, useful in applications where visibility from below is not needed, the base of the first well is formed by a porous material, such that medium can flow through the material and species diffuse within it, and so reach the underside of the cellular entity.

### Example 2a

Fig. 4i shows an embodiment in which the cellular entity holding positions can be packed more closely than in previous embodiments - this allows a lower overall volume of solution to surround them. Here the oocytes are held in microwells 152 in the substrate 150, the microwells in communication with a front side flow channel 154 and a rear side flow channel 156. The front side flow channel is defined between the substrate 150 and a removable lid 158, which when removed allows access to the wells by a pipette for adding or removing oocytes, and when in position sealed against the substrate forms a liquid-tight path from an inlet, through channel 154 to an outlet as in the embodiment in Fig. 1a. The rear channel 156 may be formed similarly between the substrate and a removable rear component 160; alternatively the rear component 160 might be bonded permanently to the substrate, or the channel 156 might be formed within the substrate itself. The rear channel might flow between an inlet and outlet as shown, or may just lead to a single connection through which fluid can flow in either direction.

In use the embodiment in Fig. 4i operates as previously described: oocytes are added or removed by pipette with lid 158 removed; flow of suspension solution through the channels 162 in the base of the wells is controlled by the pressure in the channel 156. When the lid 158 is replaced solution can be flowed through the channel 154. In order to expel air from the channel, it is necessary for the advancing meniscus in the channel to contact the liquid in the wells. This can be arranged either by appropriate design of the wells, so that the meniscus sits at the top of the well, or by use of slight backpressure in the rear side channel to position the meniscus so that the liquid front will contact it. The microwells can be formed in the substrate by appropriate means known in the art of microfabrication, such as micromoulding, microembossing, laser drilling, photolithographic patterning followed by wet or dry chemical etching. The microwells might be formed in the material of which the substrate is made, for example by laser drilling a pattern of holes in the portion of a polymer component. Alternatively, the microwells might be formed in a separate component which is then mounted in the substrate component 150. In this case advantageous fabrication method are for example etching wells in a silicon substrate, patterning and forming an array of wells in a photo-patternable material such as the photoexpoxy SU8, or similar known means.

### Example 2b

Figs. 4j, 4k and 4l show a further embodiment with similar features to that in fig. 4i, except that here the channels 162 lead laterally from the base of the wells, allowing clearer visibility of the oocytes from below. Fig. 4k shows a plan view in which the parts are numbered as in fig. 4i. Fig. 4j shows a cross section at AA in fig 4k, through the oocyte wells, and fig. 4l shows a cross section at BB, showing how the channels 162 intersect the rear side flow channel 156.

### Example 2c

Fig. 4m shows a further embodiment in which the oocyte wells 152 which are formed in the substrate 150 comprise a constriction within them that acts to retain the oocytes. The wells act like storage capillaries and when filled with medium, retain the medium by means of surface tension. The substrate 150 can be formed from hydrophilic material (e.g. acrylic) and typical diameters of the wells will be smaller than 1mm, and so capillary retention will be effective. The oocytes can be retained in their wells by simply covering the top and bottom surfaces of the substrate with covers166, 168. This forms a very simple and easy-to-handle means of transporting the oocytes. If the oocytes are to be perfused with medium then the covers are replaced with flow lids 158, 160 as shown in fig. 4n. Liquid flowed through the inlet channel 158 then contacts the menisci in the wells and allows medium to flow through the wells. Contact of the menisci and expulsion of air from the inlet channel 158 can be assisted by applying a pressure from the rear side to move the meniscus to the top of the wells 152.

In use, oocytes are pipetted into the wells from above and move with the pipetted liquid or settle by gravity onto the location position. The wells are shown as completely full in figs. 4m and 4n but the pipette volume could be chosen to keep the meniscus close to, but not exactly at, the end of the well. The oocytes can be observed from above, parallel to the axis of the wells, if visibility is satisfactory. Alternatively the wells can be observed from the side. The apparatus can be operated on its side to enable a standard inverted microscope to be used, and then moved back to vertical to allow pipette access. If side visibility is needed, the wells should be arranged as a 1D array; if visibility from the top is adequate then they can be arranged in 2D. In either case, the small size of the wells and their close arrangement means that a large number of oocytes can be cultured in a relatively small apparatus. The system in which the apparatus is used is envisaged as comprising a computer which will record which oocytes require intervention following inspection. The correct action on each can then be taken automatically once the substrate is returned to pipetting orientation.

Figure 4o shows a further embodiment in which the oocytes are positively retained in individual locations when the flow channel lid is in place. In this embodiment the lid 158 comprises a front side flow channel 154 which communicates with one or more channels 174, spaced to align with the wells 152. The diameter of the channel is smaller than that of the oocyte, so preventing the oocyte from entering the channel 154 under conditions of reverse flow or under gravity if the combination of lid 158 and substrate 150 are tilted or turned upside down. In this embodiment the rear side channel 156 is formed as part of the substrate 150 rather than by contact with a separable component. This embodiment is suitable for an oocyte transportation device which allows perfusion of the oocytes with medium.

It is clear that any of the embodiments of the invention may accommodate more than one oocyte or other cellular entity in a given well, if individual identification of the oocytes is not required. The embodiments in figs. 4m, 4n and 4o are particularly suited for this. The wells 152 may be made of a similar diameter to the oocytes so that these are retained in sequence. The well might be of uniform diameter, or of greater diameter at the mouth and with a narrower base as in fig. 4a, of similar diameter to the oocytes. In the case that the well mouth is of similar dimension to the oocytes, the pipettor will not be accommodated within the well, and so can be arranged to seal to the surface of the substrate 150 around the mouth during the pipetting process.

### Example 3

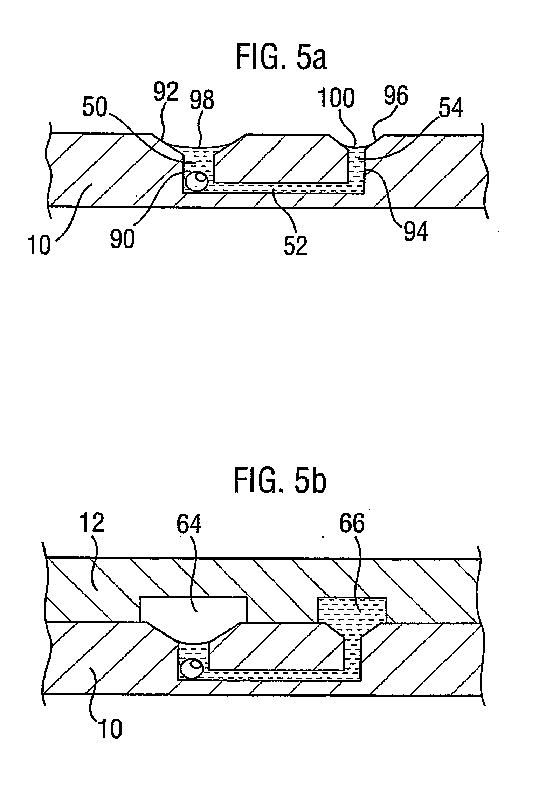

Figs. 5a - 5b show an alternative embodiment of the invention. In Fig. 5a a first well 50 has a base portion 90 of diameter chosen to accommodate the cellular entity and a wider mouth portion 92 which acts to accommodate pipetting of liquid into the well. A channel 52 communicates with a second well 54, again with a base portion 94 and a mouth portion 96. In Fig. 5b the substrate is shown fitted with a lid 12 comprising a first and a second channels 64 and 66. These channels flow from an inlet well to an outlet well in the manner of Fig. 1a. In each of the wells, a capillary stop exists at the junction between the wider mouth portion and the narrower base portion. The material of the substrate is arranged to be hydrophilic, so there is a greater pressure across a meniscus in a narrower channel than in a wider one: therefore a low pressure will suffice to move the meniscus in the wider portion, but not in the narrower.

In use, oocytes are pipetted into the first well along with a measured amount of medium. Liquid flows by capillary action through the channel 52 to the second well 54. The second well is narrower than the first, and so will fill by capillary action to a meniscus 100 at the point where the narrow base meets the broader mouth. The meniscus 98 in the first well will be at or above the capillary stop position in the first well. The oocyte is observed from below. When liquid around the oocyte needs to be exchanged, a lid 12 is fitted and liquid flowed through the channel 66. When this contacts the meniscus 100 the capillary stop in well 54 is broken. The pressure in channel 66 is then lowered and liquid will flow from the first well to the second until the meniscus in the first well reaches the start of the narrow base portion, which narrowing acts as a capillary stop, given the pressure in channel 66 is less than the pressure across the meniscus at the capillary stop position. Liquid added to the first well via the channel 64 will contact the meniscus and the capillary stop will be broken: liquid can then flow through the first well via the second to the outlet channel 66 until the meniscus once again reaches the capillary stop position.

The capillary stop feature of this embodiment may also be achieved using a change in the contact angle of the liquid to the wall of the well, as is known in the art, which may be used in conjunction with or instead of a change in diameter. The lower portion of the well is made hydrophilic and the upper portion less hydrophilic, or hydrophobic: the well will then tend to empty under negative pressure from the channel 52 (and if the upper portion of the first well 50 is hydrophobic, positive pressure across the meniscus in this portion) until the meniscus reaches the start of the hydrophilic portion, whereupon the contact angle will decrease, the pressure across the meniscus will increase, and given correct design of the channel 52 and the second well 54, flow will cease.

The embodiment in figs. 5a and 5b is fabricated, for example, by standard plastic moulding processes. The base portion 90 of the first well is small: preferably 500 microns or less. This base portion might be moulded, or might be drilled, for example by a laser drilling process. The second well 54 might in practice be any structure that exerts a capillary force on liquid within it, for example a capillary channel, a shallow chamber within the substrate, or an absorbent material placed within a well or chamber of larger minimum dimension. The substrate might be formed from a single material or more than one, in particular if a different contact angle is required in the upper part of the first well, this could be formed from a different plastic bonded to that of the lower portion; alternatively surface coatings or treatments could be used to control the contact angle in the two portions. The lower part of the wells could be formed in a subcomponent of the substrate, by microfabrication, for example by photopatterning of SU8 photoepoxy; the subcomponent would then be mounted into a plastic moulding which comprises the upper part of the well.

### Example 4

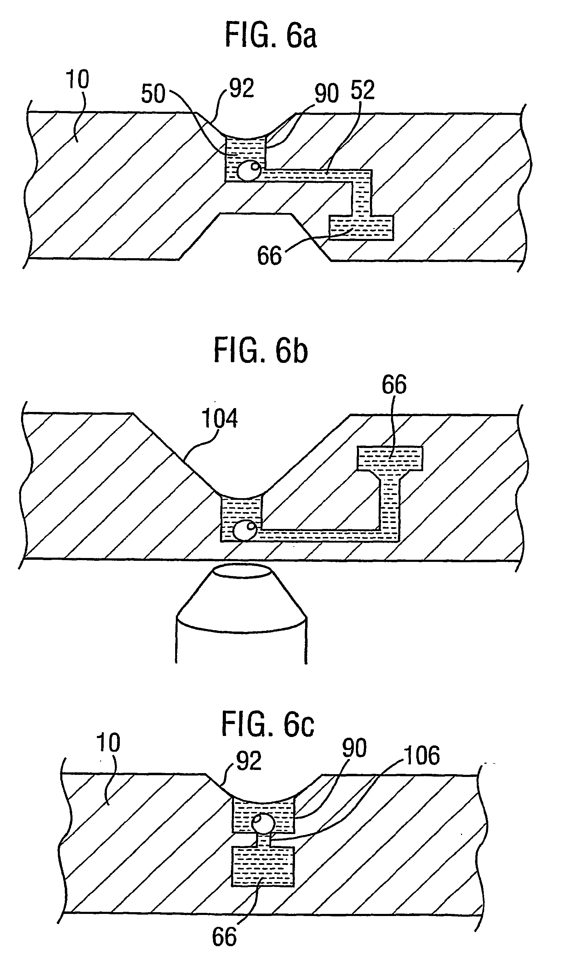

In the previous embodiments the apparatus comprised a lid to provide flow paths to the wells in the substrate. Figs. 6a and 6b show embodiments in which liquid supply to the wells is by sequential pipetting, with liquid flow through the wells achieved by means of channels as in the previous embodiment. In these embodiments a removable lid is no longer necessary - the first wells can be left open and accessible for pipetting at all stages of the process. The substrate 10 comprises one or more outlet channels 66 communicating with the well channels 52. A slight negative pressure is applied to the liquid in channel 66, this pressure being less than that needed to force liquid past the capillary stop in the inlet well. For each aliquot pipetted into the well, liquid will flow from the first well into the channel 66 until the meniscus reaches the capillary stop, whereupon flow will stop. Flow through the well is controlled by the pipetting action. In this way, if the pipetted volume is greater than that of the base portion 90, the oocyte is bathed in whichever solution was last pipetted. If the pipetted volume is smaller, then mixed solutions will coexist in the base portion and will mix slowly by diffusion. To achieve a gradient in concentration with time in this system, it is necessary to rely on diffusive mixing within the well (which will expose the oocyte to a concentration gradient from top to bottom, and so might be undesirable) or to mix remotely a series of aliquots of gradually changing concentration and pipette them in sequence into the well. In the embodiments in figs. 6a and 6b the oocyte is held at a position at the base of the well. The same principles apply to the embodiment in Fig. 6c, where the oocyte is held at a constriction 106 in the base portion of the well.

### Example 5.

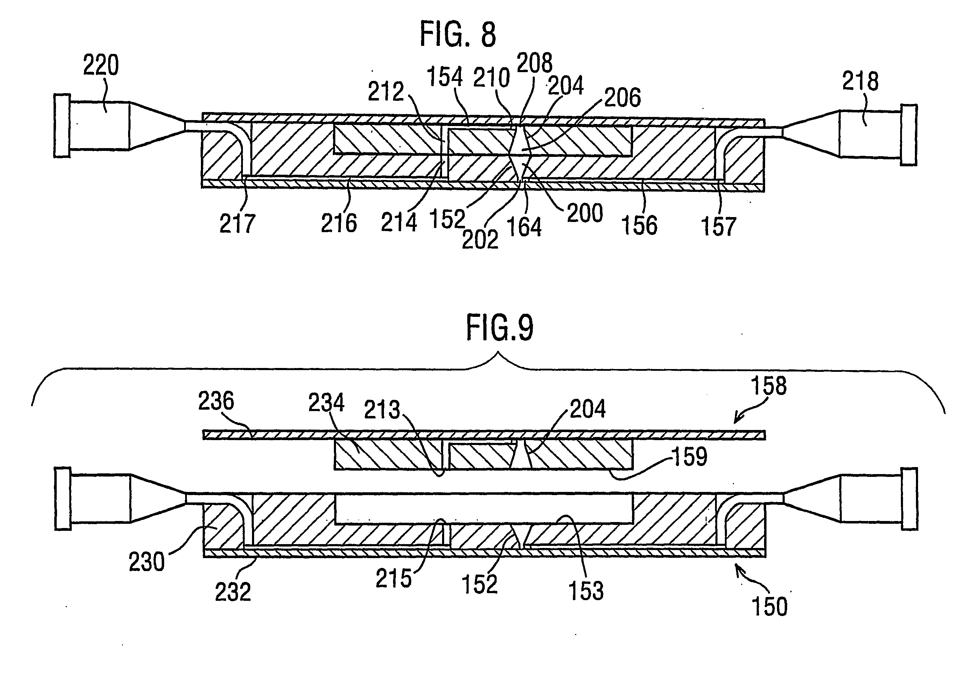

Figures 8 and 9 show a further embodiment of the type shown in figure 4o, in which one or more cellular entities such as oocytes, embryos or other cells are positively retained in an individual location on a substrate when the flow channel lid is in place. For simplicity the apparatus of Figure 8 shows a single retention location but it will be understood that the invention encompasses apparatus comprising a plurality of such retention locations. The apparatus comprises a substrate (referred to in this example as a base component) 150 and a lid component 158, each comprising a flow system, and so arranged as to fit together to complete a flow path through the oocyte location, and to be separable in order to load oocytes into the apparatus or remove them from it. The base component has a base sealing surface 153 and the lid has a lid sealing surface 159, which when brought into proximity act to complete one or more fluidic pathways between the base and the lid. Figure 8 shows the lid assembled onto the base. Figure 9 shows the lid displaced upwards from the base to show the appearance of the two components when the apparatus is disassembled. Common numerals refer to common parts in figure 4o and figures 8 and 9.

The base component 150 includes one or more first flow systems, each comprising a well 152 opening to a base sealing surface 153, the well adapted to contain one or more oocytes, a channel 156 extending from the well, the channel being adapted to prevent the oocyte from leaving the well, the channel being in fluid communication with a port 157, optionally via one or more further channels. A fluidic connection 218 is optionally provided as part of the base component to facilitate fluidic communication between the port 157 and an external flow system. Alternatively, the fluidic connection might be associated with an appliance in or on which the base component is located. The well 152 might be of uniform cross-sectional dimensions throughout its depth. The well optionally comprises a wider opening 200 and a narrower inner region 202, the opening being so sized as to permit entry of a pipette to dispense and/or aspirate one or more oocytes into/from the well, and the narrower region 202 so sized as to locate the oocyte in a defined position. The channel 156 may have a constriction 164 located and so sized as to prevent the oocyte from leaving the well.

Optionally, and as shown in Figures 8 and 9, one or more second flow systems are included in the base component, each comprising a channel 214 that extends to a port 215 on the base sealing surface 153, the channel 214 being in fluid communication with a further port 217, optionally via one or more further channels 216. A fluidic connection 220 is optionally provided as part of the base component to facilitate fluidic communication between the port 217 and an external flow system. Alternatively, the fluidic connection might be associated with an appliance in or on which the base component is located.

The lid component 158 includes one or more flow systems, each comprising a channel 204, analogous to the channel 174 in the embodiment of figure 4o, that is brought into fluid communication with the well 152 when the lid is fitted to the base, the channel 204 being in fluid communication with an outlet port 213 defined in the lid. The flow system optionally comprises one or more further channels 154 extending from the channel 204, forming a fluid pathway extending to a port 213. One or both of the channel 204 and the channel 154 are adapted to prevent the oocyte from leaving channel 204. A fluidic connection (not shown in figures 8 and 9) is optionally provided as part of the lid component to facilitate fluidic communication between the port 213 and an external flow system. Alternatively, the fluidic connection might be associated with an appliance in or on which the lid component is located.

The embodiment in Figures 8 and 9 has a preferred arrangement whereby the port 213 is located on the lid sealing surface 159, and a fluidic connection to the flow system in the lid is made via a second flow system included in the base component 150 as described above, this second flow system in the base component having a first port 215 defined in the base sealing surface 153, and defining a fluidic pathway extending from the port 215 to a second port 217. The flow systems in the lid and base are so arranged that when the lid is assembled onto the base, the base sealing surface 153 and the lid sealing surface 159 are brought into proximity and channel 212 is brought into fluid communication with the second flow system in the base via ports 213 and 215. This second flow system provides fluidic communication between the port 217 and the port 213 in the lid, and hence to the well 152.

The channel 204 might be of any uniform cross-sectional dimension. In the preferred embodiment shown in figures 8 and 9 the channel 204 is tapered and of circular cross section, with approximately the same diameter opening as that of the well 152.

One or both of the channel 204 and the channel 154 might be sized to prevent the oocyte from passing through the fluidic path from the well 152 to the external connection. Optionally, as shown in figures 8 and 9, a constriction 210 is provided in the channel 154, which acts to prevent passage of the oocyte along the channel 154.

The embodiment in Figures 8 and 9 shows the fluidic connections 218 and 220 as Luer-type, with a tubular portion abutting the ports 157 and 217. These connections may take any form suitable to communicate between the flow systems of the apparatus and any external flow systems or appliance used to provide fluid flow to or from the apparatus. For example, any or all ports might be brought into fluid communication with ports associated with an external appliance in any way known in the art. The apparatus might be designed to interfit with an appliance in such a way as align the ports with ports located on the appliance, so allowing fluid to be moved between them.

The base 150 and lid 158 might be provided with features that cause them to be retained together when they are assembled. Such features might be snap-fit features, or other clip-like features that are formed as part of the base, lid or both. Alternatively a separate clip or mounting device might be provided to retain the base and lid together. A plurality of base and lid components might be accommodated in a common mounting device, for example to allow them to be handled as a group. This embodiment might be particularly advantageous to allow a number of smaller devices to be arrayed in a format compatible with standard robotic fluid handling apparatus.

The sealing surfaces 153 and 159 might be planar or might have features that assist closure of fluidic pathways through them when they are held in proximity. Such features might include indentations or keying features that act to hold the surfaces together or in alignment in the plane parallel to the surfaces. Preferably at least one of the surfaces is formed from a compliant material, disposed either over the whole surface or only in the vicinity of the ports. Features such as raised portions encircling ports in one or both of the surfaces are preferably provided in order to seal with a higher pressure in these regions for a given force used to hold the surfaces together.

The apparatus can be fabricated by various methods as known in the art. The embodiment shown in figures 8 and 9 has a design that allows a wide range of materials and fabrication methods to be used. A particularly advantageous feature is that if transparent materials are used the oocyte is readily observable when located in either the well 152 or the channel 204, especially if the oocyte is located in the inner region 202 of the well, in which case the oocyte can be observed through the solid base of the well. In the design in Figures 8 and 9 this base can have optical properties that can be chosen or controlled easily in the fabrication process. The design allows observation using lens objectives that can be matched to the optical properties of the solid well base material, and which is not done through a depth of liquid medium of variable and uncertain properties, through a filter medium or against a filter medium, with uncertain contrast.

The base component of the embodiment in Figures 8 and 9 is shown as comprising a body part 230, in which are defined components of the flow systems, including wells, channels, constrictions, ports and features facilitating external fluidic connection. The components open to the major surfaces of the body part will be in the form of open channels or other features. The body part is sealed to a planar component 232, which acts to close the flow components formed on the lower surface of the body component. Similarly, the lid component is shown as comprising a body part 234, closed with a planar component 236. A preferred fabrication method is to form the body components from moulded PDMS and the planar components 232 and 236 from glass, then using plasma activation of one or both joining surface to achieve a bond. Such an assembly method is well known in the art. Alternative fabrication methods are known, for example using a plastic planar closure component, either with a modified plasma bonding process or using a force to hold the body part and planar part together, for example by clamping or clip-fitting. Plastic formation methods such as injection moulding or embossing might be used to form one or both components. While the embodiment in figure 8 shows the channels and other flow components formed in the body components 230, 234, in alternative embodiments one or more flow components might be formed wholly or partially in the planar components 232, 236. Typical dimensions of the fluidic features of the apparatus are chosen to be appropriate for the type of cellular entity to be handled and the intended application. In some applications a single cellular entity or a small group of cellular entities are required to be held in individual wells, to achieve an individual chemical environment for each, or to place them in an easily locatable X-Y position. Examples of such applications occur in embryology, e.g. handling of oocytes and embryos, in which individual control of the chemical environment might be desirable and is it useful to be able to observe numbers of oocytes or embryos in-situ in the apparatus using rapid movements of an X-Y stage. In such applications the inner region of the wells will be sized to retain only a single, or a small number of cellular entities. Therefore a cross-sectional dimension of the inner region of the well is preferably in the range 1 to around 10 times the maximum cross sectional dimension of the entity, more preferably between 1 and 5 times the maximal dimension. For example, for use with oocytes and embryos of maximal dimension 100 um, the inner region of the well preferably has a dimension in the range from 100 um to 1 mm, more preferably 100 um to 500 um. For culture or handling of single mammalian cells, or small groups of cells, the well dimension will preferably be in the range 10 um to 100 um, more preferably 10 um to 50 um. In other embodiments part of the inner region of the well has a cross-sectional dimension that is less than the maximum dimension of the cellular entity.

The wells, and other features of the flow systems, can have any cross-sectional profile appropriate to the application and fabrication method. In particular, for ease of fabrication the wells may have circular cross-section. The channels may have an approximately rectangular cross-section if formed by moulding or embossing from a machined mould, or may have a rounded profile if etched from a solid substrate using, for example, microengineering methods as used in standard microfabrication processes such as photolithography / film deposition / etching processes as known in the art.

The minimum dimension of a channel leading from the well, or a constriction in the channel if one is present, is chosen to be sufficient to retain the cellular entity against movement with flow of liquid through the well and channel, with allowance made for any tendency of the cellular entity to deform under pressure. A preferred minimum dimension of the constriction is of order half the minimum dimension of the entity.

Example dimensions for the embodiment of Figures 8 and 9, for use with oocytes and embryos, are as follows (all dimensions are approximate). The well 152 is of circular cross-section, 3 mm diameter at the opening 200 defined at the base sealing surface 153, tapering to 500 um diameter at the inner region 202. The well is around 3 mm deep. The channel 204 is also in the form of a tapered well of circular cross-section, 3 mm diameter at the opening 206 defined at the lid sealing surface 159 and 1 mm diameter at inner region of the channel near the junction with the channel 154. The constrictions 164 and 210 are 50 um high.

An example of the fluidic connections and operation of the apparatus is given below. Other forms of connection and operation are possible and within the scope of the invention.

The apparatus is first disassembled to give access to the well 152. The well 152 is then part-filled with medium through the port 157, using fluidic connection 218, via channel 156. One or more oocytes are pipetted into the well, and the lid 158 assembled onto the base 150. The fluidic path through the apparatus can then be filled via port 157, while the oocyte or oocytes are retained in the region in the fluid pathway between the constrictions 164 and 210, i.e. in the space defined by the well 152 and the channel 204. Medium can then be flowed through the well in either direction. The oocyte can be retrieved from the well by pipetting from the well 152 with the lid removed as in previous embodiments. Flow through channel 156 might be enabled to assist the process of aspiration.In operation it is advantageous to achieve complete filling of the apparatus with liquid without trapping of air bubbles, and to clear bubbles from the system effectively if they should be introduced. The arrangement of Figures 8 and 9, in which the fluidic pathway when the apparatus is assembled comprises a tapered well 152 adjoining a tapered channel 204, is particularly preferred in this respect as it has been found in experiments that this arrangement gives better priming and subsequent clearing of bubbles that other arrangements, such as wells with square bases, or pathways in the assembled apparatus which have overhangs or sudden changes of diameter. For this reason, designs where the openings 200 in the well 152, and 206 in the channel 204, are the same cross-sectional shape are preferred. The present embodiment in which the openings of the well 152 and channel 204 are of circular cross-section and the same diameter is particularly preferred.

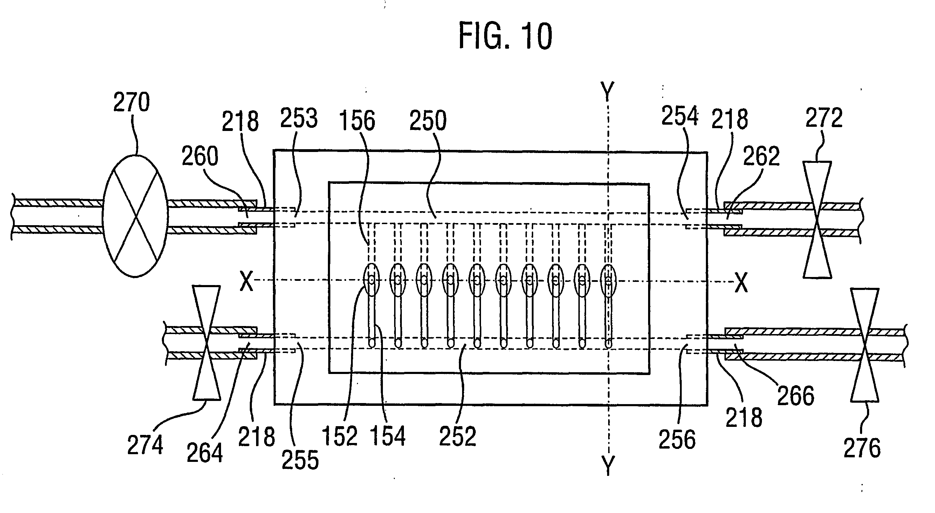

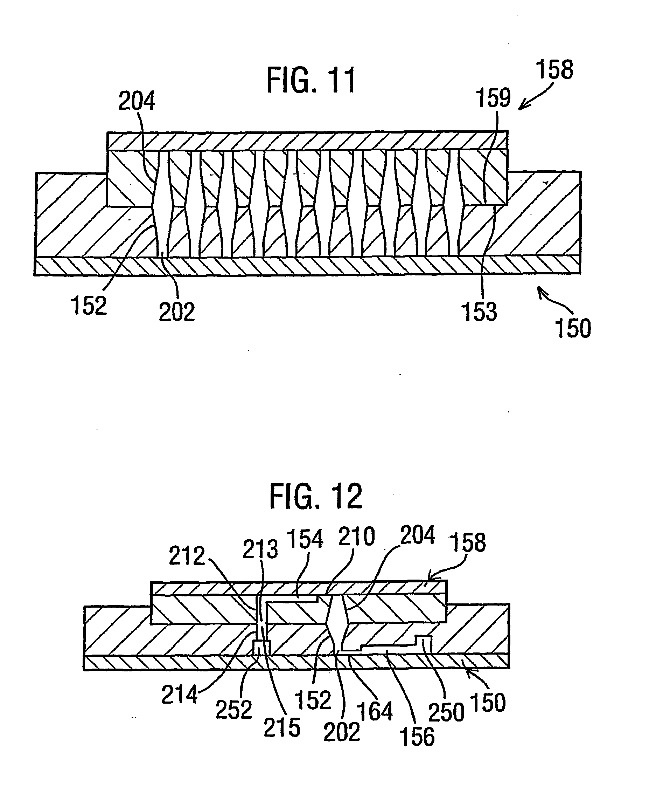

Figures 10, 11 and 12 show a further embodiment, in which a plurality of wells are provided, each associated with a flow system as described for the embodiment in Figures 8 and 9, with further channels and ports arranged to provide fluidic connection to the plurality of wells in parallel. The arrangement of the flow systems associated with the wells is similar to that shown in Figures 8 and 9 and the same features are indicated with the same numerals. Figure 10 shows a plan view of the apparatus when assembled, Figure 11 a cross-section at X-X and Figure 12 a cross-section at Y-Y.

The apparatus comprises a base component 150 and a lid component 158, which can be assembled together to form a closed fluidic path through the apparatus from an inlet port to an outlet port, and can be disassembled to give access to the well 152. The base component has a base sealing surface 153 and the lid has a lid sealing surface 159, which when brought into proximity act to complete a number of individual fluidic pathways between the base and the lid. The base component includes a plurality of flow systems, each comprising a well 152 opening to the base sealing surface 153 and at least one channel 156 extending from the well, with an optional constriction 164 in the channel that prevents the oocyte from leaving the well. The channels 156 are in fluid communication with a first manifold channel 250 that extends to one or more ports 253, 254, each in fluid communication with a fluidic connection 218, which port or ports act to allow fluid flow to and from a flow system external to the apparatus. In figure 10 the fluidic connection is shown as a tube 218 which abuts the ports 253, 254 but any suitable flow connection known in the art may be used, such as the Luer flow connection shown in the embodiment in Figures 8 and 9.

The embodiment in Figure 10 has a similar preferred arrangement to that in Figures 8 and 9, whereby the fluidic connections are made via the base component 150. Therefore the base component also includes a second flow system comprising a plurality of channels 214, each extending to a port 215 defined at the sealing surface 153 of the base component, the channels 214 being in fluid communication with a second manifold channel 252. The second manifold channel 252 extends to one or more ports 255, 256, each in fluid communication with a fluidic connection 218, which ports act to allow fluid flow to and from a flow system external to the apparatus.

The lid component 158 includes a plurality of flow systems each comprising a channel 204 opening to the lid sealing face 159, the channel optionally being in the form of a tapered well as shown in figures 11 and 12, the channel 204 being in fluid communication with a port 213 defined in the lid. The flow system optionally comprises one or more further channels 154 forming a fluid pathway extending to a port 213. Either the channel 204 or the channel154 are adapted to prevent the oocyte from leaving channel 204, for example by provision of a constriction 210 in the channel at the junction with the well.

In the preferred embodiment shown in Figures 10, 11 and 12, fluidic connection is made to the flow systems in the lid via the second flow system in the base, and a further channel 212 is provided in the flow system in the lid which communicates with the channel 204 and opens to a port 213 defined in the lid sealing surface 159. The flow systems in the lid and base are so arranged that when the lid is assembled onto the base, the base sealing surface 153 and the lid sealing surface 159 are brought into proximity and channel 212 is brought into fluid communication with channel 214 in the second flow system in the base via ports 213 and 215. This second flow system provides fluidic communication between the second manifold channel 252 and channel 204, and hence to the upper opening of well 152.

When the apparatus is assembled, the opening to each well 152 is brought into fluid communication with the opening to each channel 204, and the channel 212 in the lid is brought into fluid communication with the channel 214 in the base, for each of the flow systems. This establishes a closed fluidic path through each well, from the first manifold channel 250, through the channel 156, the constriction 164 (if present), the well 152, the channel 204 in the lid, the constriction 210 (if present) in the lid, the channels 154 and 212, the channel 214 and the second manifold channel 252.

Embodiments are included in the invention in which fluidic communication with the channel 204 of each flow system is achieved via one or more fluidic connections associated with the lid component. In this case the second flow system in the base, discussed above, can be omitted. This might involve fluidic connection to each flow system in the lid independently, via ports 213 located in the lid. Embodiments are included in the invention in which the second manifold channel 252 is formed in the lid component. In this case one or more fluidic connections may be made to the second manifold channel via fluid connectors associated with the lid. Alternatively, connecting channels might be provided in the lid and the base, in fluid communication with each of the ends of the second manifold channel in the lid which, when the apparatus is assembled, align and establish fluid communication in the manner described above for the individual flow systems.

The first and second manifold channels may be of uniform cross-sectional dimensions along their length or these may vary along their length. The flow systems including the wells 152 and communicating channels may be essentially similar to one another or may be designed to be different. Each flow system appears in parallel with the others, communicating between the first and second manifold channels. When filled with liquid, the flow through each of the flow systems will be proportional to the liquid flow resistance through each. Advantageously the apparatus is designed to have similar liquid flow resistance through each flow system, as measured between the ports at the ends of the manifold channels. If the apparatus is designed so that the flow resistance in the manifold channels is negligible compared with the flow resistance through each of the flow systems, then by designing each flow system to be similar the flow through each will be similar. If however there is significant flow resistance in the manifold channel, then either the dimensions of the manifold channels will be designed to vary along their lengths, or the dimensions of the flow systems will be designed to differ from one another, in order to make similar the total flow resistance and hence the liquid flow through each of the wells. In certain cases it may be advantageous to set the flow through the wells to be unequal, in which case the dimensions of the manifold channels and / or flow systems can be set accordingly.

The embodiment of figures 10, 11 and 12 can be fabricated by the same means as used for the embodiment in figures 8 and 9 or for any previous embodiment. Similar variations in the location of channels and other flow features are within the scope of the invention. The dimensions of features in the embodiment in Figures 10, 11 and 12 will be similar to those specified for the embodiment in Figures 8 and 9.

An example of the fluidic connections and operation of the apparatus is given below. Other forms of connection and operation are possible and within the scope of the invention.

If two or more connections are provided to the first manifold channel, as shown in figure 10, then one connection (260) will function as a fluid inlet and a second (262) as a vent. Further connections may be provided to act as further inlets if it is required to introduce different liquid media into the apparatus. In some embodiments only one fluid connection will be made to the first manifold channel, in which case it will act as a fluidic inlet and venting will be achieved through the flow systems comprising the wells and communicating channels. Preferably fluid connections (264, 266) are provided at least at the extremities of the second manifold channel. In normal use these act as fluidic outlets. A valve 272 is provided to open and close the vent 262 and valves 274 and 276 are provided for outlets 264, 266. A pump 270 is provided to actuate fluid flow into the inlet 260.

In use, the apparatus is first disassembled to give access to the wells 152. The first manifold channel 250 is flushed with medium by means of the pump 270 with the vent valve 272 open. Valve 272 is then closed and wells 152 are part-filled using the pump with medium through the first manifold channel. Oocytes are then pipetted into the wells and the lid component fitted. With valves 274 and 276 open and valve 272 closed, actuating the pump will fill the remainder of the flow systems. The provision of outlet ports at both extremities of the second manifold channel 252 allows air to be displaced by liquid in both directions and avoids the situation which arises with only one outlet, of the trapping of air by a liquid slug located in the second manifold channel between the air and the outlet. The second manifold channel fills with liquid to both outlets, and then one of the valves, 274 say, is closed and liquid will flow through the system via the other outlet port and outlet valve 276. More than two outlets may be provided at points along the length of the second manifold channel if needed to achieve optimum venting.

The embodiment in Figures 10, 11 and 12 shows a number of flow systems connected in parallel between the first and second manifold channels. It will be appreciated that other forms of fluidic connection are included in the scope if the invention. For example, a series connection of all or some of the flow systems of an apparatus is envisaged. Also, further first and second manifold channels may be provided, each pair communicating with further groups of flow systems. Distribution channels may be provided as part of the apparatus, which communicate with the further first and second manifold channels in order to provide fluid flow to and from them. These further channels may be formed wholly within the base component, wholly within the lid component, or may be formed and closed between them when they are assembled. They may pass between the base and the lid components by means of ports brought into fluid communication in the manner described above.

Fluid reservoirs may be incorporated in the apparatus according to any of the embodiments above, acting to supply fluid to or receive fluid from any or all of the wells and flow systems included in the apparatus. These reservoirs may be located in the base component or the lid component, or a further component adapted to interfit with the base or lid component to give fluidic connection with them. Pumps and valves may also be provided in the apparatus, either as part of the base, the lid or further component. A component comprising fluid reservoirs, valves and pumps may be designed to be removable from the apparatus, or vice-versa, without disassembling the lid from the base.

The apparatus of the invention may be designed to operate together with an appliance that provides and controls fluid flow to and from the apparatus. The appliance may house one or more such apparatus. The appliance may supply liquid to, or receive liquid from the apparatus, or may act on the apparatus to produce fluid flow to or from reservoirs on the apparatus, for example by applying gas pressure to a liquid reservoir on the apparatus, or physical force to a deformable portion of the apparatus to induce fluid flow within it. The apparatus may comprise further components such as electrically powered or controlled components, such as pumps, valves, measuring instruments and temperature controllers, and so may be provided with electrical connections to make contact with corresponding connections on the appliance. The apparatus might locate on or to the appliance so as to bring fluidic ports into communication and / or electrical connections into contact when the apparatus is so located. The apparatus may be provided with electrical power sources that might store power to run such components when the apparatus is detached from the appliance. Advantageously the apparatus is designed to be operable with further, portable power supplies and external control means, to allow it to operate for periods independently of the appliance, and is capable of being used to ship the cellular entities within it between one appliance and another, located remotely from the first.

A further method of unloading the oocyte from the well is useable when the channel 204 in the lid is in the form of a well large enough to accommodate a pipette. The oocyte can be moved between the well 152 and the channel 204 by flow or, if the apparatus is inverted while still assembled, the oocyte will sediment from the well into the channel. The base 150 is then removed from the lid leaving the channel 204 open to allow access with a pipette through the opening 206. The oocyte is then aspirated from the inner region 208 of the channel 204 into the pipette. The embodiment shown in Figures 8 and 9 is suitable for this further method of unloading, when adapted so that the channel 204 is in the form of a well, having a larger diameter in its inner region 208 than the diameter of the inner region of the well 152. This allows easier pipetting out of the channel 204 in the lid than out of the well 152, whilst still giving an enclosed environment in the inner region of well 152 for control of exposure to media or precise location of the oocyte for easy optical observation. In some embodiments the second well 204 may have an inner region large enough to allow the pipette to reach the bottom of the well. In other embodiments, the second well may be dimensioned to locate the pipette at a distance above the bottom in order to control the approach of the pipette tip to the oocyte or ooctyes. The channel or well 204 may then be used for handling of moving oocytes, and if a plurality of such wells are provided, may be adapted for use with robotic pipetting equipment in the same way as has been described for the wells 50, 152 in previous embodiments.

The invention provides in one aspect devices having channels closed along their length by the surface of one substrate meeting the other. The invention provides in another aspect devices where channels are formed within either or both of two substrate, and are brought into engagement such that aligned ports, openings or wells provide a fluidic pathway, with sealing surfaces being provided to seal around the ports openings or wells. In either case lid substrate and base substrate join to form a complete a fluidic pathway.

## Claims

1. A device for culturing and/or maturing a cellular entity, comprising a substrate (10, 150) having one or more wells (50) open to a first major surface of the substrate, said one or more wells being adapted to hold a cellular entity, and one or more fluidic channels (52) open to the one or more wells, the device being configured to allow exchange of liquid in the well, **characterized in that** the well is adapted to allow dispense and removal of the cellular entity using dispensing means, and the device is adapted to prevent the cellular entity from leaving the one or more wells through the one or more fluidic channels.

2. A device as claimed in claim 1 in which the one or more fluidic channels (52) is sized to prevent a cellular entity from leaving the one or more well through the fluidic channel.

3. A device as claimed in any preceding claim including retaining means (164) to retain the cellular entity against movement with the flow of liquid through the well and fluid channel.

4. A device as claimed in claim 3 in which the retaining means comprises a constriction.

5. A device as claimed in claim 4 in which the constriction is provided in the well.

6. A device as claimed in claim 5 in which the constriction is provided in the fluidic channel.

7. A device as claimed in any preceding claim in which the one or more wells is tapered to become smaller with distance from the major surface.

8. A device as claimed in any preceding claim in which the one or more well is partially defined in a layer of porous material.

9. A device as claimed in any preceding claim in which the one or more fluidic channels is formed by laminating a layer of porous material between two non-porous layers.

10. A device as claimed in any preceding claim in which a dimension of the one or more wells is less than or equal to 3 mm.

11. A device as claimed in claim 10 in which a dimension of the one or more wells is in the range 10 microns to 1 mm.

12. A device as claimed in any preceding claim in which the one or more fluidic channel has a minimum dimension of the order of half the minimal dimension of the cellular entity.

13. A device as claimed in any preceding claim in which the one or more fluidic channels is provided with a gas-permeable region (78) for allowing removal of gas from the fluidic channel when being filled with a liquid.

14. A device as claimed in any preceding claim in which said one or more fluidic channels is also open to one or more second wells in said major surface of said substrate.

15. A device as claimed in any preceding claim in which the one or more fluidic channels is provided adjacent the base of the one or more wells, and a further fluid channel (64) is provided adjacent and in fluid communication with the top of the well.

16. A device as claimed in any preceding claim in which at least one of said one or more fluidic channels is formed between the major surface of a further substrate (158) and a major surface of said substrate (150), the further substrate being releasably secured to said substrate.

17. A device as claimed in claim 15 including clamping means for holding the first and second substrate in alignment and bringing them into engagement with one another.

18. An apparatus for culturing and/or maturing cellular entities including: a device as claimed in any preceding claim, means to provide fluid flow to and from the device, and control means to control the fluid flow.

19. Use of a device as claimed in any preceding claim in a method of culturing or maturing one or more cellular entities.

20. A method of culturing and/or maturing a cellular entity, the method comprising:
dispensing one or more cellular entities (14) into one or more wells (50) of a device as claimed in any one of claims 1 to 18,
flowing a liquid medium into the one or more wells through said one or more fluidic channels (52), and
culturing or maturing the cellular entity in the liquid medium in the device.

## Patentansprüche

1. Vorrichtung zur Kultivierung und/oder Reifung einer zellularen Entität, die ein Substrat (10, 150) umfasst, das eine oder mehrere Mulden (50) aufweist, die zu einer ersten Hauptfläche des Substrats hin offen sind, wobei die eine oder mehreren Mulden dafür ausgelegt sind, eine zellulare Entität aufzunehmen, und einen oder mehrere fluidische Kanäle (52) aufweist, die zu der einen oder den mehreren Mulden hin offen sind, wobei die Vorrichtung dafür konfiguriert ist, den Austausch von Flüssigkeit in der Mulde zu gestatten, **dadurch gekennzeichnet, dass** die Mulde dafür ausgelegt ist, das Abgeben und Herausnehmen der zellularen Entität unter Verwendung eines Abgabemittels zu ermöglichen, und die Vorrichtung dafür ausgelegt ist zu verhindern, dass die zellulare Entität die eine oder mehreren Mulden durch den einen oder die mehreren fluidischen Kanäle verlässt.

2. Vorrichtung nach Anspruch 1, wobei der eine oder die mehreren fluidischen Kanäle (52) so bemessen sind, dass eine zellulare Entität daran gehindert ist, die eine oder mehreren Mulden durch den fluidischen Kanal zu verlassen.

3. Vorrichtung nach einem der vorangehenden Ansprüche, die ein Haltemittel (164) enthält, um die zellulare Entität gegen eine Bewegung mit der Strömung von Flüssigkeit durch die Mulde und den Fluidkanal zu sichern.

4. Vorrichtung nach Anspruch 3, wobei das Haltemittel eine Verengung umfasst.

5. Vorrichtung nach Anspruch 4, wobei die Verengung in der Mulde angeordnet ist.

6. Vorrichtung nach Anspruch 5, wobei die Verengung in dem fluidischen Kanal angeordnet ist.

7. Vorrichtung nach einem der vorangehenden Ansprüche, wobei die eine oder mehreren Mulden so verjüngt sind, dass sie mit zunehmender Entfernung von der Hauptfläche kleiner werden.

8. Vorrichtung nach einem der vorangehenden Ansprüche, wobei die eine oder mehreren Mulden teilweise in einer Schicht aus porösem Material definiert sind.

9. Vorrichtung nach einem der vorangehenden Ansprüche, wobei der eine oder die mehreren fluidischen Kanäle durch Laminieren einer Schicht aus porösem Material zwischen zwei nicht-porösen Schichten gebildet werden.

10. Vorrichtung nach einem der vorangehenden Ansprüche, wobei eine Abmessung der einen oder mehreren Mulden maximal 3 mm beträgt.

11. Vorrichtung nach Anspruch 10, wobei eine Abmessung der einen oder mehreren Mulden im Bereich von 10 Mikron bis 1 mm liegt.

12. Vorrichtung nach einem der vorangehenden Ansprüche, wobei der eine oder die mehreren fluidischen Kanäle eine Mindestabmessung in der Größenordnung der Hälfte der kleinsten Abmessung der zellularen Entität aufweisen.

13. Vorrichtung nach einem der vorangehenden Ansprüche, wobei der eine oder die mehreren fluidischen Kanäle mit einer gasdurchlässigen Region (78) versehen sind, um das Abziehen von Gas aus dem fluidischen Kanal zu ermöglichen, wenn er mit einer Flüssigkeit gefüllt wird.

14. Vorrichtung nach einem der vorangehenden Ansprüche, wobei der eine oder die mehreren fluidischen Kanäle außerdem zu einer oder mehreren zweiten Mulden in der Hauptfläche des Substrats hin offen sind.

15. Vorrichtung nach einem der vorangehenden Ansprüche, wobei der eine oder die mehreren fluidischen Kanäle neben der Basis der einen oder mehreren Mulden ausgebildet sind und ein weiterer Fluidkanal (64) neben und in Strömungsverbindung mit der Oberseite der Mulde angeordnet ist.

16. Vorrichtung nach einem der vorangehenden Ansprüche, wobei mindestens einer der einen oder mehreren fluidischen Kanäle zwischen der Hauptfläche eines weiteren Substrats (158) und einer Hauptfläche des Substrats (150) ausgebildet ist, wobei das weitere Substrat lösbar an dem Substrat befestigt ist.

17. Vorrichtung nach Anspruch 15, die Klemmmittel aufweist, um das erste und das zweite Substrat in Ausrichtung aufeinander zu halten und miteinander in Eingriff zu bringen.

18. Apparatur zur Kultivierung und/oder Reifung zellularer Entitäten, die Folgendes enthält: eine Vorrichtung nach einem der vorangehenden Ansprüche, Mittel zum Erzeugen einer Fluidströmung zu und von der Vorrichtung, und Steuermittel zum Steuern der Fluidströmung.

19. Verwendung einer Vorrichtung nach einem der vorangehenden Ansprüche in einem Verfahren zum Kultivieren oder Reifen einer oder mehrerer zellularer Entitäten.

20. Verfahren zum Kultivieren und/oder Reifen einer zellularen Entität, wobei das Verfahren Folgendes umfasst:
Abgeben einer oder mehrerer zellularer Entitäten (14) in eine oder mehrere Mulden (50) einer Vorrichtung nach einem der Ansprüche 1 bis 18,
Einleiten eines flüssigen Mediums in die eine oder mehreren Mulden durch den einen oder die mehreren fluidischen Kanäle (52), und
Kultivieren oder Reifen der zellularen Entität in dem flüssigen Medium in der Vorrichtung.

## Revendications

1. Dispositif de culture et/ou de maturation d'une entité cellulaire, comprenant :
un substrat (10, 150) doté d'un ou de plusieurs puits (50) ouverts sur une première surface principale de substrat, ledit ou lesdits puits étant adaptés pour contenir une entité cellulaire, et
un ou plusieurs canaux (52) de fluide ouverts sur le ou les puits,
le dispositif étant configuré pour permettre un échange de liquide dans le puits,
**caractérisé en ce que**
le puits est adapté pour permettre de placer et d'enlever l'entité cellulaire en utilisant un moyen de placement et
le dispositif est adapté pour empêcher que l'entité cellulaire quitte le ou les puits par le ou les canaux de fluide.

2. Dispositif selon la revendication 1, dans lequel le ou les canaux de fluide (52) sont dimensionnés de manière à empêcher qu'une entité cellulaire quitte le ou les puits par le canal pour fluide.

3. Dispositif selon l'une quelconque des revendications précédentes, comprenant des moyens de retenue (164) qui empêchent l'entité cellulaire de se déplacer avec l'écoulement de liquide qui traverse le puits et le canal pour fluide.

4. Dispositif selon la revendication 3, dans lequel le moyen de retenue comprend un étranglement.

5. Dispositif selon la revendication 4, dans lequel l'étranglement est prévu dans le puits.

6. Dispositif selon la revendication 5, dans lequel l'étranglement est prévu dans le canal pour fluide.

7. Dispositif selon l'une quelconque des revendications précédentes, dans lequel le ou les puits se rétrécissent à une distance croissante de la surface principale.

8. Dispositif selon l'une quelconque des revendications précédentes, dans lequel le ou les puits sont définis partiellement dans une couche de matériau poreux.

9. Dispositif selon l'une quelconque des revendications précédentes, dans lequel le ou les canaux pour fluide sont formés en stratifiant une couche de matériau poreux entre deux couches non poreuses.

10. Dispositif selon l'une quelconque des revendications précédentes, dans lequel une dimension du ou des puits est inférieure ou égale à 3 mm.

11. Dispositif selon la revendication 10, dans lequel une dimension du ou des puits est de l'ordre de 10 micromètres à 1 mm.

12. Dispositif selon l'une quelconque des revendications précédentes, dans lequel le ou les canaux pour fluide ont une dimension minimale de l'ordre de la moitié de la dimension minimale de l'entité cellulaire.

13. Dispositif selon l'une quelconque des revendications précédentes, dans lequel le ou les canaux pour fluide sont dotés d'une partie (78) perméable au gaz qui permet la sortie du gaz hors du canal pour fluide lorsqu'il est rempli d'un liquide.

14. Dispositif selon l'une quelconque des revendications précédentes, dans lequel le ou lesdits canaux pour fluide sont également ouverts sur un ou plusieurs deuxièmes puits prévus dans ladite surface principale dudit substrat.

15. Dispositif selon l'une quelconque des revendications précédentes, dans lequel le ou les canaux pour fluide sont prévus en position adjacente à la base du ou des puits, un autre canal (64) pour fluide étant prévu en position adjacente et en communication d'écoulement avec le sommet du puits.

16. Dispositif selon l'une quelconque des revendications précédentes, dans lequel au moins l'un desdits canaux pour fluide est formé entre la surface principale d'un autre substrat (158) et une surface principale dudit substrat (150), l'autre substrat étant fixé de manière libérable audit substrat.

17. Dispositif selon la revendication 15, comprenant des moyens de passage qui maintiennent le premier et le deuxième substrat en alignement et les amènent à s'engager l'un sur l'autre.

18. Appareil de culture et/ou de maturation d'entités cellulaires, comprenant un dispositif selon l'une quelconque des revendications précédentes, des moyens qui assurent un écoulement de fluide vers le dispositif et hors de ce dernier et des moyens de contrôle qui contrôlent les commandes de fluide.

19. Utilisation d'un dispositif selon l'une quelconque des revendications précédentes dans un procédé de culture ou de maturation d'une ou plusieurs entités cellulaires.

20. Procédé de culture et/ou de maturation d'une entité cellulaire, lequel procédé comprend les étapes qui consistent à :
placer une ou plusieurs entités cellulaires dans un ou plusieurs puits (50) d'un dispositif selon l'une quelconque des revendications 1 à 18,
faire écouler un milieu liquide dans le ou les puits par ledit ou lesdits canaux (52) pour fluide, et
cultiver ou faire mûrir l'entité cellulaire dans le milieu liquide à l'intérieur du dispositif.
